# EUROPEAN PATENT APPLICATION

(11) **EP 4 059 445 A1**
(43) Date of publication of application: **21.09.2022**
(21) Application number: 21315040.2
(22) Date of filing: 18.03.2021
(51) Int. Cl.: A61B 17/00, A61M 37/00

(54) **MEDICAL IMPLANT AND CATHETER DEVICE FOR A MEDICAL IMPLANT**

(71) Applicant: HoliStick Medical, 75003 Paris (FR)
(72) Inventor: Spencer, Andrew, Boston, MA 02113 (US); Josephson, John, Watertown, MA 02472 (US); Roche, Ellen, Galway (IE); Warnack, Boris, 4104 Oberwil (CH); Pouletty, Philippe, 75005 Paris (FR); Pau, Antoine, 75005 Paris (FR); Gard, Marco, 10031 Borgomasino (IT)
(74) Representative: Hepp Wenger Ryffel AG

(57) **Abstract**

The invention relates to a medical implant (1) comprising a patch (2). The implant is adapted to close a defect (3), preferably a an opening in a ventricular or atrial septum (4). The medical implant (1) comprises at least one mechanical anchor (5). The anchor may comprise a barb (6) and is adapted to pierce the patch (2) and/or biological tissue (4) such as to fix the medical implant (1) to said tissue (4). The medical implant can be deployed by a delivery device (7). The delivery device (7) may comprise a balloon (8', 8"), preferably at least two balloons (8', 8").

## Description

The present invention relates to a medical implant and a catheter device for a medical implant according to the preamble of the independent claims.

Defects in tissue, for example atrial septal defects (ASD) or ventricular septal defects (VSD), are a fairly common condition in humans that is typically treated minimally invasively. Such defects can cause a variety of symptoms such as shortness of breath, stroke, and a higher burden on the heart and lungs.

As a consequence, a myriad of implantable devices has been proposed in the prior art, many of which can be deployed in a minimally invasive way.

For example, closure of atrial septal defects (ASD) by deploying umbrella-like implants through a catheter have been disclosed by Lock et al. (DOI: 10.1161/01.CIR.79.5.1091).

In particular, adhesive patches have been proposed to close such defects. For example, the copending unpublished application PCT/IB2019/001058 discloses several adhesive patches adapted to close defects in a human body.

EP 2 665 504 discloses deployable barbed microneedle arrays for use with tissue.

However, known implants have several disadvantages. In particular medical implants that comprise adhesive patches may need long surgical interventions to ensure safe attachment to a tissue wall, may require more complex interventions, and are more prone to operator error. Longer treatments lead to higher risks associated with the treatment.

Some implants are known that are attachable by exerting a mechanical force against a tissue (e.g. pinching). Such treatments may lead to complications and follow-up treatments, for example due to inflammation and/or necrosis and/or accidental perforation.

Thus, the object of the present invention is to overcome the drawbacks of the prior art, in particular to provide a medical implant and a catheter device for delivering an implant that provides easy, quick and safe attachment of an implant to tissue and reduces risks for a patient.

This and other objects are achieved by a medical implant and a catheter device according to the characterizing portion of the independent claims of the invention.

The medical implant according to the invention preferably comprises a patch and is adapted to close an opening. The opening may be an opening in a ventricular or atrial septum. Preferably, the opening is a defect in a tissue wall, such as patent foramen ovale (PFO). Alternatively, the opening may also be a left atrial appendage. The medical implant comprises at least one mechanical anchor. The mechanical anchor may comprise at least one barb.

The mechanical anchor is adapted to puncture at least one of the patch and biological tissue such as to mechanically fix the medical implant to said tissue. The medical implant is further adapted to be deployable by a delivery device. The delivery device may comprise a balloon. Preferably, the delivery device comprises at least two balloons.

At least one, preferably all, mechanical anchors may comprise at least one barb, preferably a plurality of barbs. Barbs provide particularly safe attachment. Barbs may in particular be bio-inspired barbs, i.e. shaped according to naturally occurring barbs. For example, mechanical anchors comprising barbs may be modelled after cacti needles or porcupine quills. Particularly preferably, three-dimensional data representing the shape of a naturally occurring spine/quill may be used to produce a mechanical anchor, for example by 3D-printing, template molding, or lithography.

The implant according to the invention may also be used for closure of the left atrial appendage and/or aneurysms.

A mechanical anchor typically denominates an element that provides a mechanical connection to tissue. Preferably, the mechanical connection extends to the medical implant, i.e. the mechanical anchor directly connects, and provides attachment, between the implant and the tissue. In alternative embodiments, however, it is also possible that the mechanical anchor is mechanically attached to tissue and provides a separate attachment means to the implant, for example via magnetic forces.

The mechanical anchor typically has an elongated and in particular straight shape with a sharp tip in order to puncture tissue efficiently. In some embodiments, the mechanical anchor may also comprise a head portion which has a larger cross-section in a plane perpendicular to a longitudinal axis of the mechanical anchor than the body of the mechanical anchor. Such a head portion can limit the movement of the mechanical anchor in a direction along its longitudinal axis, in particularagainst going through the implant entirely, in which case the attachment may be insufficient.

The mechanical anchor may be sized such as to not entirely penetrate a typical tissue wall. Typically, the mechanical anchor has a length of less than 5 mm, in particular of about 0.5 to 5 mm, preferably 0.5 to 2 mm, particularly preferably 0.7-1 mm. The diameter of the mechanical anchor, in particular of its body portion, is preferably in the range of 0.5 to 1.5 mm.

The anchors may be macroanchors with a size, in particular a length along a longitudinal axis, in the range of 0.5-5 mm.

Additionally or alternatively, the anchors may be microanchors with a size in the range of 1-1000 pm, preferably 50-400 µm. Preferably, the medical implant comprises a plurality of anchors, in particular a plurality of microanchors. The density of anchors on the medical implant surface may be in the range of 100-5000 anchors/cm², preferably 200-2000 anchors/cm², particularly preferably 300-700 anchors/cm².

In preferred embodiments, the mechanical anchor comprises at least one barb. Barbs may preferably be angled with respect to a longitudinal axis of the mechanical anchor. The barbs may be angled at an angle in the range of 0 to 90°, preferably 0-80°, particularly preferably 10-70°. The barb may also be angled with respect to the longitudinal axis at an angle of 10-20°, 20-30°, 30-40°, 40-50°, 50-60°, and/or 60-70°. It will be understood that all barbs may be angled at the same angle or at different angles from any range disclosed herein. The barbs may, additionally or alternatively, be interpenetrating. The barb provides a particularly simple and secure way of attaching the mechanical anchor to tissue. If barbs are present, they typically extend away from the body of the mechanical anchor pointing in a direction towards the base portion of the mechanical anchor. In other words, the barb points away from the direction of penetration in a tissue. Thus, the barb does not provide substantial resistance against penetration of a tissue, but prevents or at least hinders removal of the mechanical anchor. Additionally or alternatively, the mechanical anchor may comprise one or a plurality of grain spike-like barbs.

The mechanical anchor is preferably such that a head area of the mechanical anchor is arranged in contact with on outer surface of a balloon when arranged on a delivery device in order to transmit a force. Preferably, the mechanical anchor pierces the medical implant such that a portion of the body, in particular a portion comprising barbs, is arranged on a different side of the implant than the head area.

A patch is a particularly easy element that provides closure to a tissue opening.

It is possible that the implant comprises multiple patches. For example, two patches may be arranged on either side of a tissue opening, wherein at least one patch is attached or attachable to the tissue by means of a mechanical anchor. The second patch may be connected to the first patch via a suture, a wire, or another adaptable interconnecting strut. It is also possible that the second patch is directly attached to the tissue wall via mechanical connectors as well.

In a particularly preferred embodiment, the patch comprises or consists of a mesh-like material. To this end, at least two, preferably at least three, mechanical anchors may be linked to each other by means of said mesh-like material. The mesh-like material provides mechanical flexibility and thus easy deployment. The mesh may be adapted to, once implanted, facilitate and enable tissue ingrowth. The formed tissue makes the mesh impermeable to blood and thus closes the opening. Additionally, the mesh may be bio-erodible and degrade in the human body after formation of tissue.

The mechanical anchors may be oriented with an angle between slightly larger than 0° and 90° with respect to a surface of the medical implant.

The mechanical anchors may comprise of a bioabsorbable material. Preferably, the mechanical anchors consist of a bioabsorbable material.

Bioabsorbable materials degrade in the human body over a certain time frame, preferably within a year. It is known in the art, however, to tune the degradation rate such that a material degrades in the human body in any period of time, for example one week, one month, six months, or multiple years.

Degradable mechanical anchors may soften or entirely disintegrate over time, thus reducing potential for inflammation. In the case of ASD closure, degradable anchors may enable re-crossing of the septum with minimal risk of embolism during the re-crossing.

In particular, magnesium alloys are biodegradable in the human body. Additionally or alternatively, polymers may also be used. Alternatively, the mechanical anchors may comprise a biologically non-absorbable material. The mechanical anchors may also consist of a biologically non-absorbable material.

Particularly suited materials are titanium, titanium alloys, implant steel, or non-absorbable polymers.

A biologically non-absorbable material may be advantageous if permanent mechanical attachment is desired. For example, mechanical anchors may be the only attachment mechanism, or they may provide additional safety to an adhesive attachment.

At least one mechanical anchor may comprise a polymeric material. The mechanical anchor may also consist of a polymeric material. In particular, the polymeric material may be an absorbable polymer such as an absorbable polyester. Particularly suited polymeric material are lactide-based absorbable polymers and/or co-polymers, in particular a polymeric material selected from poly(lactic-co-glycolic acid) and poly(L-lactic acid). Preferably, the polymeric material is degradable with a degratdation time of 3 months to 18 months, preferably about one year, in the human body.

The mechanical anchor may comprise a metallic material. Preferably, the mechanical anchor consists of a metallic material.

The metallic material may be absorbable or non-absorbable. For example, the mechanical anchor may comprise titanium or titanium alloys such as nitinol, implant-grade steel, magnesium alloys, tantalum or tantalum alloys.

The implant may comprise an adhesive composition. The adhesive composition may preferably be curable by exposure to electromagnetic radiation.

Examples of adhesive compositions and implants comprising such adhesive compositions are described in PCT/IB2019/001058, which is incorporated here by reference.

Mechanical anchors are particularly suited as a temporary and/or additional fixation for implants that comprise an adhesive composition because the mechanical anchors can be quickly deployed and provide secure attachment while the adhesive composition cures. In particular, mechanical anchors comprising a bioabsorbable material may be used in this context. Such mechanical anchors may provide temporary attachment while the adhesive composition is being cured. After completion of the curing process and/or healing process, the mechanical anchors may no longer be necessary and can thus degrade in the human body. Alternatively, it is also possible to configure the anchors to remain in the body to provide additional local attachment and thus safety against detachment, while a sealing attachment across the entire opening may be provided by the adhesive.

At least one of the mechanical anchor and the implant may comprise a magnetic element. The magnetic element is adapted to connect the medical implant and the mechanical anchor at least partially via a magnetic force.

For example, the mechanical anchor may comprise a permanent magnet in a head area. The implant may comprise a ferromagnetic material, for example metallic fibers woven into the patch in an area intended to be connected to the mechanical anchor. Alternatively, the medical implant may comprise a permanent magnet that provides attachment to a ferromagnetic mechanical anchor.

It is also conceivable to use a permanent magnet in both the mechanical anchor and the medical implant. By configuring the magnetic poles appropriately, it is possible to predetermine the orientation of the medical implant at the implantation site, for example.

Magnetic elements may be beads with a permanent magnetic moment, fibers of a magnetic material, or elements with other shapes mechanically attached to the medical implant and/or the mechanical anchor.

It is also conceivable to configure the entire patch or the entire medical implant to consist of a magnetic material, or a material that is attracted by a magnet (i.e. a ferromagnetic material) .

Ferromagnetic materials are known in the art. Particularly preferred ferromagnetic materials are also biocompatible and/or biodegradable, for example iron-based alloys.

The mechanical anchor may comprise at least one cavity.

The cavity may be configured as a bore, a round cavity, or as any other hollow shape inside the mechanical anchor. Particularly preferably, the mechanical anchor is configured as a hollow shape.

Preferably, the surface of the at least one mechanical anchor comprises micro-sized holes that are connected to the at least one cavity.

Thus, the micro-sized holes may provide a fluid communication channel between the cavity and an area outside the mechanical anchor.

Preferably, a releasable substance is disposed in the at least one cavity of at least one mechanical anchor. The releasable substance may in particular be releasable through the micro-sized holes.

The mechanical anchor may additionally comprise a mechanism to selectively release the releasable substance. For example, the mechanical anchor may comprise an element in the cavity that swells in the presence of water and thus creates a pressure in the cavity once implanted, causing a release of the encapsulated substance.

However, preferably, the releasable substance is released passively via diffusion.

The releasable substance may comprise an adhesive composition. The adhesive composition may be adapted to be curable by exposure to electromagnetic radiation.

Particularly preferably, the releasable substance is a photopolymerizable adhesive adapted to be crosslinked after release from the cavity. The mechanical anchor, in particular a microanchor and/or macroanchor with or without barbs, in this case facilitates delivery of the adhesive to within the tissue before photopolymerization.

An adhesive composition released from a cavity in the mechanical anchor may provide additional attachment of the mechanical anchor to a tissue. It is also possible to provide two-component adhesives with components in different cavities, such that the two components are mixed upon release.

In particular if the mechanical anchor does not comprise barbs, such an adhesive composition may be advantageous. However, it is possible to use a releasable substance comprising an adhesive composition in mechanical anchors with barbs as well.

The person skilled in the art will understand that the adhesive composition comprised in the releasable substance may be the same or a different composition used in the medical implant.

Additionally or alternatively, the releasable substance may comprise a pharmacologically active substance, in particular an anti-inflammatory substance.

The releasable substance may reduce a body response in the tissue (for example, immune response, inflammation, or similar) to the inserted mechanical anchor and/or to the erosion of a polymeric material (of the mechanical anchor, patch material, or other).

The medical implant may comprise thrombogenic elements.

Thrombogenic elements may be used to form a thrombus at or near the implant. Thus, it is possible to fill a larger opening with a relatively small implant because the formed thrombus may assist occlusion of the opening.

A thrombus may also provide an additional adhesive force between the tissue and the medical implant.

Preferably, the thrombogenic elements comprise fibers, in particular microfibers.

Fibers may comprise or consist of poly(ethylene terephthalate), thermoplastic polyurethanes, polypropylene, and/or polyethylene.

The medical implant may comprise a self-expanding support structure.

The self-expanding support structure may be a mesh or a skeleton made of a shape memory material, for example a shape memory polymer or a nitinol alloy.

The self-expanding structure may, in particular, be the mechanical anchor and provide mechanical attachment by pressing against a tissue wall after expansion. Alternatively, the self-expanding structure may be used in addition to other mechanical anchors.

The mechanical anchors may be adapted to reduce their length upon exposure to humidity.

For example, this may be achieved using a polymeric material with a mechanical stress in the material. Exposure to liquid may swell the polymer and activate and/or enable diffusion, such that the polymer may relax the mechanical stress by shrinking, in particular along the longitudinal axis. The material may also be adapted such that diffusion is additionally only activated and/or enabled at or above a minimum temperature, for example body temperature.

A reduction in size may increase the attachment force provided by the mechanical anchors, in particular if the mechanical anchor comprises barbs. The mechanical anchor can be pierced into the tissue to attach the medical implant. After piercing, the length of the mechanical anchor may be reduced to the humidity, and thus the anchors exert a higher force on the implant/tissue, as shrinkage along the longitudinal axis may provide a pulling force on the barbs.

Preferably, the medical implant comprises a force distribution structure. The force distribution structure may consist of a bio-erodible material. The force distribution structure is connected to the mechanical anchors such that it distributes a mechanical pressure applied to the medical implant to the at least one mechanical anchor, in particular amongst at least two mechanical anchors, particularly preferably amongst a plurality of macroanchors and/or microanchors.

For example, the force distribution structure may be a plate, a grid, a strut, or a plurality of struts. The force distribution structure is functionally adapted to at least partially redirect a force applied anywhere on the force distribution structure to the mechanical anchors attached to it.

Preferably, the force distribution structure comprises or consists of a metallic and/or polymeric material.

The at least one mechanical anchor may comprise at least one needle, in particular a micro-needle.

A needle substantially functions like a barb. In addition, a needle may be hollow and assist the release of a releasable substance. Thus, the releasable substance may be distributed more evenly in the tissue surrounding the mechanical anchor.
The mechanical anchor may comprise or consists of an adhesive material.

A mechanical anchor comprising or consisting of an adhesive material may further increase the retention of the medical implant and enhance the force necessary to detach the implant from tissue. Such an implant may thus allow for a particularly safe treatment through a combination of mechanical attachment and chemical/physical adhesion.
The adhesive may preferably be pre-dried. A pre-dried adhesive is advantageous as it may provide a stiffness sufficient to at least partially penetrate a tissue wall. Absorption of water upon penetration and subsequent swelling may assist adhesion in the tissue and/or provide flexibility to adapt to a tissue wall.

Additionally or alternatively, the mechanical anchors may comprise or consist of a photosensitive resin and/or a thermoplastic.

The invention is further directed to a catheter device. The catheter device is adapted to deploy a medical device as described herein.

Preferably, the catheter device comprises at least one balloon, particularly preferably at least two balloons.

In particular, the catheter device may comprise a mechanism to apply a mechanical force to the implant, preferably by inflation of a balloon. The mechanical force may be used to pierce a tissue by means of a mechanical anchor.

Furthermore, the catheter device may comprise a light-guiding element, for example an optical fiber, to deliver light from a light source, for example an LED.

The catheter device may comprise a connection element to attach the medical implant to a distal end of the catheter device. The connection element may comprise mechanical hooks, clamps, loops, and/or sutures or any combination thereof. The suture may in particular comprise a pre-determined breaking point.

The invention is further directed to a medical implant comprising an adhesive. The medical implant may, in particular, be any implant as described herein. The implant may preferably be a patch. The adhesive is arranged at least on a first surface of the medical implant. The adhesive is further formed in a pattern. Preferably, the pattern is a two-dimensional pattern or a three-dimensional pattern.

The pattern may provide a texture that helps retain the adhesive, in particular GelMA, during pressurization, leading to stronger adhesion.

Preferably, the pattern comprises mechanical anchors as described herein. In particular, mechanical anchors comprising or consisting of an adhesive material may be part of the pattern and comprise or consist of any of the adhesive materials described herein below.

The pattern may be non-uniform. In particular, the pattern may be printed on the implant by inkjet or extrusion printing. The pattern may also be regular, but comprise a 3D structure and/or a non-homogeneous topography.

Preferably, the adhesive composition comprises gelatin-methacryloyl (GelMA), in particular a GelMA of animal original. Particularly suited GelMAs are Fish GelMA, porcine GelMA, and bovine GelMA, i.e. GelMA processed and originating from fish and/or pigs and/or cows. GelMA originating from cold-water fish is particularly suited because of its low-temperature (in particular at room temperature) mechanical flexibility. However, any type of commercially available GelMA is suitable for the invention.

In particular, the GelMA, in particular if derived from pigs, may have a Bloom value/Bloom strength of 250 to 325. GelMA derived from fish may not have bloom strength.

Preferably, the GelMA has a molecular weight of 50 to 170 kDa.

Preferably, the GelMA may be formed by a mixture of at least two GelMAs of animal origin. Particularly preferably, the GelMA is formed by a mixture of fish GelMA and porcine GelMA.

A mixture of two GelMAs enables to combine properties of different GelMAs. For example, a mixture of (cold water) fish GelMA with porcine GelMA may yield a GelMA with the solubility of porcine GelMA and the mechanical flexibility of fish GelMA. It is also possible to gradually tune properties, for example solubility and mechanical flexibility, by choosing the ratio of different GelMAs, for example porcine and fish GelMA. Such GelMAs are known to the skilled person and commercially available.

Additionally or alternatively, it is also possible to tune properties of the GelMA by varying and/or mixing of different molecular masses.

In particular, inkjet or extrusion printing enables complex structures and patterns of adhesive to be arranged on medical implants that may otherwise be difficult to achieve due to brittleness of the adhesive composition upon drying.

Additionally or alternatively, the pattern may be molded, in particular using a silicone mold made froma 3D-printed structure.

Alternatively, it is also possible to arrange a continuous film of adhesive, wherein a pattern is created with a stamp while the adhesive is in a liquid state.

Particularly preferably, the adhesive composition is arranged in a pre-defined pattern such as to enable flexibility of the implant in certain directions. For example, the adhesive composition may be arranged as slices of a round disk. The implant may then be flexible along the axes separating the individual slices.

The pre-defined pattern may be a two-dimensional pattern, i.e. a substantially flat adhesive film with a patterned structure. Alternatively, the pattern may also be three-dimensional, i.e. also comprise a pattern along an axis perpendicular to the implant surface on which the adhesive composition is arranged.

A three-dimensional pattern is particularly advantageous as it allows for local tuning of pressure. For example, a pyramid that extends from the surface may be pressed against tissue with a higher local pressure than a flat film. Such structures may thus also enhance tissue integration through diffusion into the tissue.

The pattern may comprise at least one spike. Particularly preferably, the pattern comprises a plurality of spikes. A plurality of spikes is particularly advantageous because it allows for a local force concentration which in turn leads to better adhesion, even if the force exerted on the medical implant is comparably small. Additionally or alternatively, the pattern may include at least one or a plurality of pyramids, triangles, cubes, barbs, quills, or other shapes and combinations thereof.

In the following, the invention is described in detail with reference to the following figures, showing:
- Fig. 1:: schematically a medical implant being deployed with a catheter device.
- Figs. 2a-2b:: schematically a medical implant and a cross-section through a mechanical anchor.
- Fig. 3:: schematically a mechanical anchor.
- Figs. 4a-4b:: schematically a medical implant with biodegradable mechanical anchors.
- Figs. 5a-5c:: schematically mechanical anchors with a cavity.
- Figs. 6a-6b:: schematically mechanical anchors with a magnetic element.
- Fig. 7:: schematically a mechanical anchor in tissue.
- Fig. 8:: schematically a medical implant with a force distribution structure.
- Figs. 9a-9b:: schematically a medical implant comprising a self-expandible structure and thrombogenic elements and
- Fig. 10:: schematically a catheter device.
- Fig. 11a-11b:: patches with a patterned adhesive layer in a top view.
- Fig. 12:: a patch with an adhesive having a three-dimensional pattern.
- Fig. 13a-13d:: schematically a method of patterning an adhesive layer on a patch.
- Figs. 14a-14e:: different embodiments of patches with two-dimensional adhesive patterns.
- Fig 15:: embodiment of patch with a three-dimensional adhesive pattern.
- Fig 16a-16b:: array of microneedles and SEM of microbarb needle.

For clarity, reference signs for identical elements may be shown only once in certain figures.

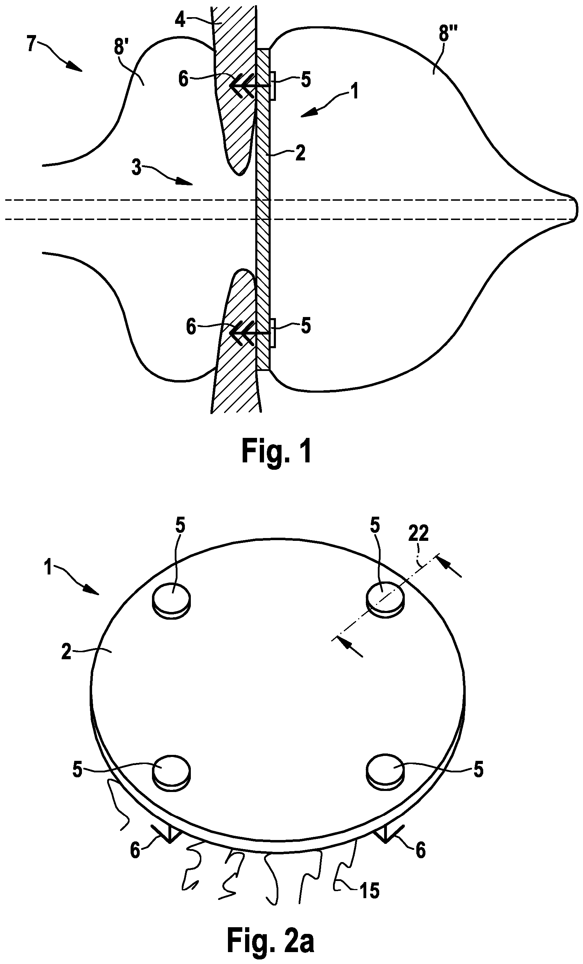

Fig. 1 shows a medical implant 1 which comprises a patch 2 that is pierced by mechanical anchors 5. The patch substantially consists of biocompatible fabric. The mechanical anchors 5 are made of a nitinol alloy and are thus biocompatible. The medical implant 1 is being deployed to a defect 3 within a tissue 4, in the present case a patent foramen ovale (PFO), by a catheter device 7 that comprises a first 8' and a second balloon 8". An anchor may be used as an alternative to the second balloon. The second balloon 8" provides a mechanical force and pushes the mechanical anchors 5 into the tissue 4. The mechanical anchors 5 further comprise barbs 6 which prevent the mechanical anchors from being removed from the tissue. Once deployed in the tissue, the mechanical anchors 5 reduce their length due to a shape memory effect of the nitinol alloy after being heated to body temperature (37°C).

Fig. 2a shows schematically a medical implant 1 similar to the one shown in Fig. 1. The medical implant 1 comprises a patch 2 of pericardium with four mechanical anchors 5 arranged evenly along a circumferential area of the patch 2. The mechanical anchors are made of a copolymer of lactic acid and glycolic acid and degrade in human body within approximately six months. The patch is equipped with thrombogenic microfibers 15. When implanted, the microfibers trigger the formation of a thrombus which provides adhesion between the patch 2 and the tissue (not shown). It will be understood that thrombogenic fibers as shown here may be combined with any implant described herein, in particular an implant with an adhesive or an adhesive pattern.

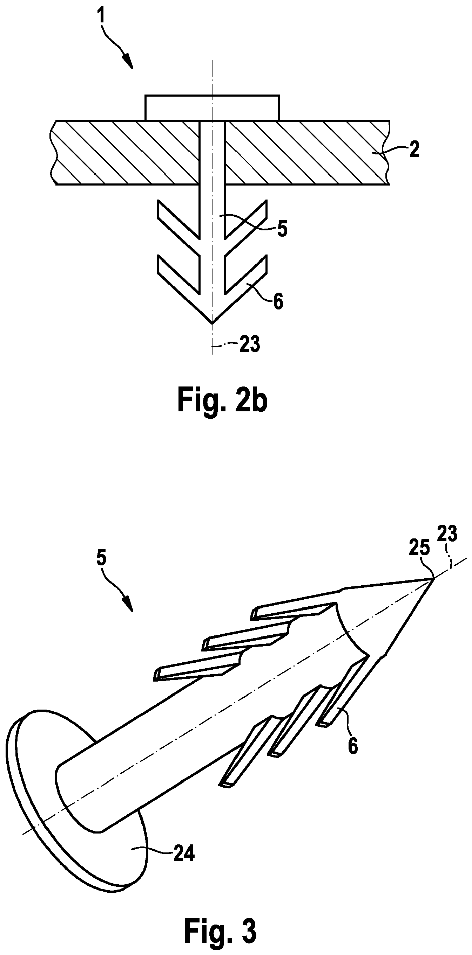

Fig. 2b shows a cross-section of the medical implant 1 along the plane 22 of Fig. 2a. The patch 2 is pierced through by the mechanical anchor 5. The mechanical anchor 5 comprises two rows of barbs 6 arranged at a distance along a longitudinal axis 23 of the mechanical anchor 5.

Fig. 3 schematically shows a mechanical anchor 5. The mechanical anchor 5 comprises two rows of three barbs 6 arranged at even distance of each other along the longitudinal axis 23 of the mechanical anchor 5. The mechanical anchor 5 further comprises a head area 24 that has a larger cross-section in a plane perpendicular to the longitudinal axis 23 of the compared to a body portion of the mechanical anchor 5. The head area 24 is adapted to hold the medical implant against accidental release in the direction of the head area 23. The barbs 6 are arranged at an angle of about 30° with respect to the longitudinal axis 23. The mechanical anchor has a sharp tip 25 adapted to pierce tissue. The shown mechanical anchor 5 is made of implant-grade steel. However, it would be possible to configure an identically shaped mechanical anchor 5 out of any suitable material disclosed herein.

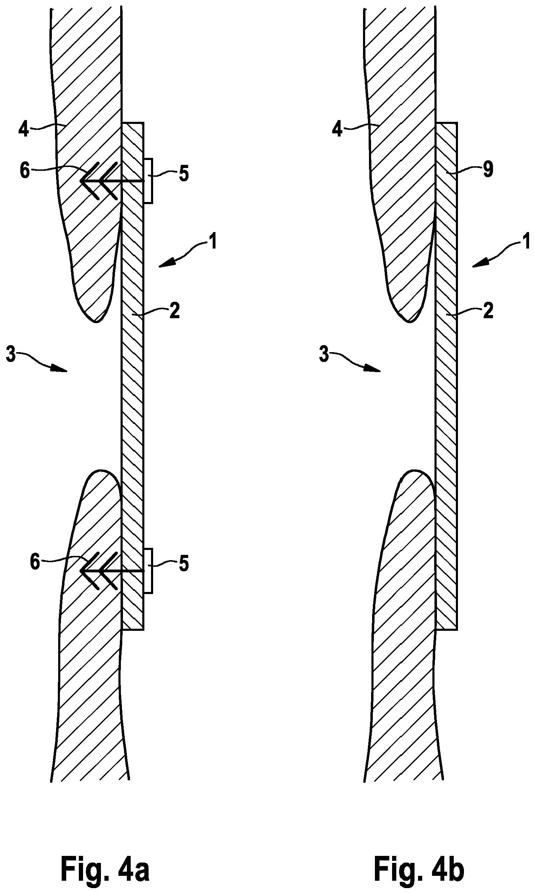

Fig. 4a shows a medical implant 1 similar to the one shown in Fig. 2a being attached to a tissue wall 4 via mechanical anchors 5 comprising barbs. The medical implant 1 closes a PFO 3. Here, the patch 2 is made of fibers that develop adhesion to the tissue 4 due to exposure to an aqueous liquid. Thus, the patch 2 is fixedly attached to the tissue 4 via adhesive forces after about 1 month. In order to keep the surgical treatment for implantation short, the medical implant 1 comprises mechanical anchors 5 made of high molecular mass poly-lactic acid. The mechanical anchors 5 reduce their length upon exposure to an aqueous liquid when at a temperature of roughly 37°C because the polymer chains are preferentially oriented along the longitudinal axis of the anchors 5. Therefore, after implantation, the mechanical anchors exert a force and push the medical implant 1 against the tissue 4. The polylactic acid degrades in the human body within 6 months. Thus, after the adhesion force has become strong enough to hold the implant, the mechanical anchors 5 start degrading.

Fig. 4b shows schematically the medical implant about one year after implantation. The mechanical anchors have degraded and are no longer present. The tissue 4 has healed and the pierced holes from the mechanical anchors have disappeared. The patch 2 now comprises an adhesive composition 9 that holds the patch on the tissue 4. The adhesive composition 9 formed via a surface reaction with blood, forming a coagulum layer that provides attachment between the tissue wall 4 and the implant 1. Additionally or alternatively, a photopolymerizable adhesive, such as GelMA, may be used.

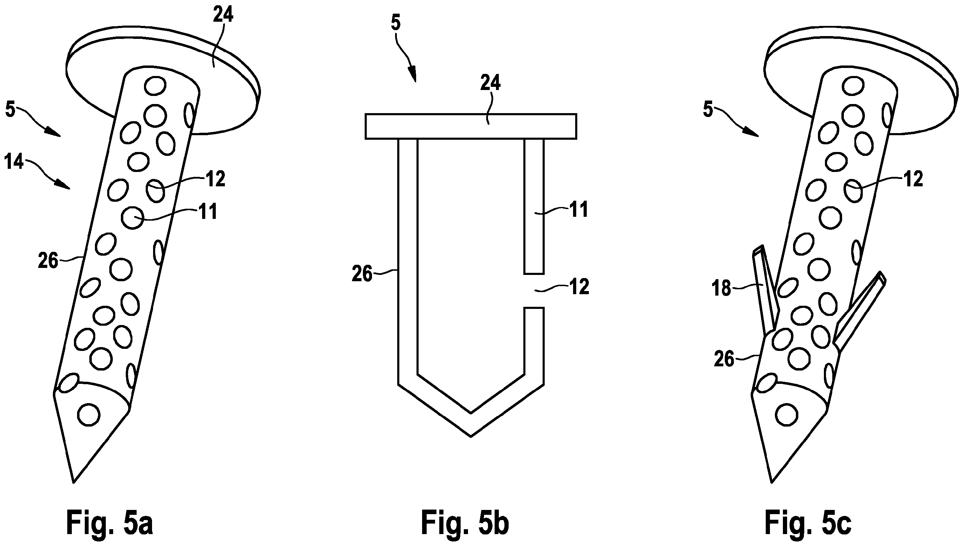

Fig. 5a shows a mechanical anchor 5 that is configured as a hollow structure closed by a head area 24. The cavity 14 formed by the hollow structure is in fluid communication with and outside area of the mechanical anchor 5 via micrometer-sized holes 12 in a side wall 26 of the anchor 5. The embodiment shown does not comprise barbs. A releasable substance comprising an adhesive 14 may thus be arranged in the cavity 11 to provide additional attachment in the tissue (not shown). The embodiment shown may thus be particularly advantageous if barbs are not suited for a particular treatment or tissue (for example, sensitive or inflammation-prone tissue).

Fig. 5b shows the mechanical anchor 5 of Fig. 5a in a cross-sectional view. The cavity 11 is delimited by the side wall 26 and the head area 24 of the mechanical anchor 5. Fluid communication between the cavity 11 and the outside area is ensured via holes 12 in the sidewall 26 of the anchor 5.

Fig. 5c shows a similar mechanical anchor 5 as shown in Fig. 5a and 5b. The embodiment here further comprises two needles 18 arranged on the sidewall 26 of the anchor 5. The needles 18 serve a double purpose. They can provide additional attachment to a tissue wall (not shown) as barbs could. In addition, the needles are hollow and provide a second fluid communication channel in addition to the holes 12. Thus, a releasable substance can be distributed in a tissue wall in an improved manner. The shown embodiment is particularly advantageous if a drug, for example an anti-inflammatory substance, needs to be released in the tissue surrounding the mechanical anchor 5. Alternatively, an adhesive may be released.

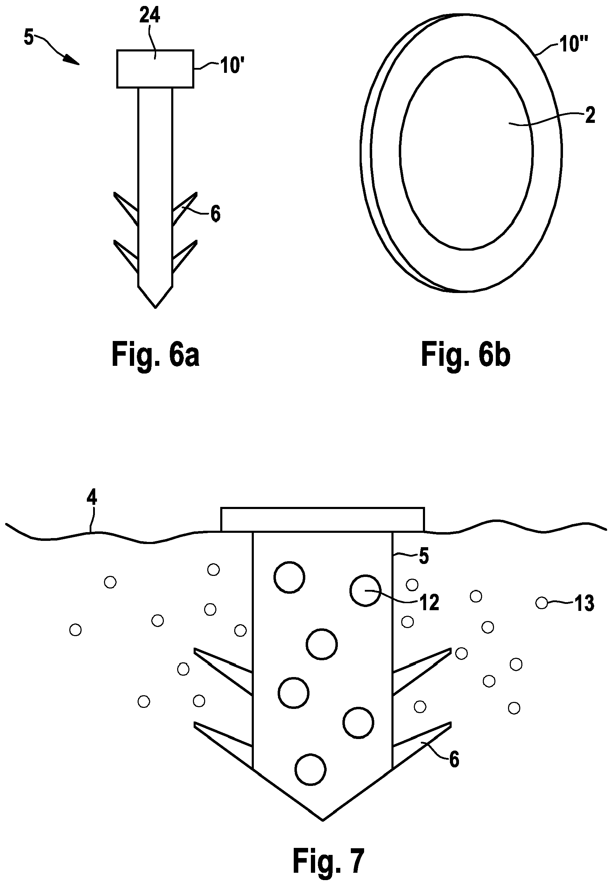

Fig. 6a shows a mechanical anchor 5 with a magnetic element 10'. The magnetic element 10' is configured as a head area 24 made of rare earth material, which may optionally be coated with an antifouling material.

Fig. 6b shows a patch 2 with a circumferential area made of a ferromagnetic iron alloy, which may optionally be coated with an anti-fouling material. It will be understood that the ferromagnetic material shown here may be replaced with a rare earth magnet as described in Fig. 6a, which in turn may comprise a ferromagnetic material in this case, while providing the same functionality.

The mechanical anchor 5 of Fig. 6a is thus adapted to be attached to the patch 2 of Fig. 6b via a magnetic force between the magnetic elements 10',10". Thus, the mechanical anchors may be anchored in a tissue (not shown) first, and the patch 2 may be inserted subsequently and attached via a magnetic force.

Fig. 7 shows a mechanical anchor 5 with a cavity (not visible, see Figs. 5a and 5b, for example) which is inserted in a tissue wall 4. The mechanical anchor 5 is held in the tissue 4 by barbs 6. The cavity is filled with an anti-inflammatory drug 13 that is released through holes 12 into the tissue.

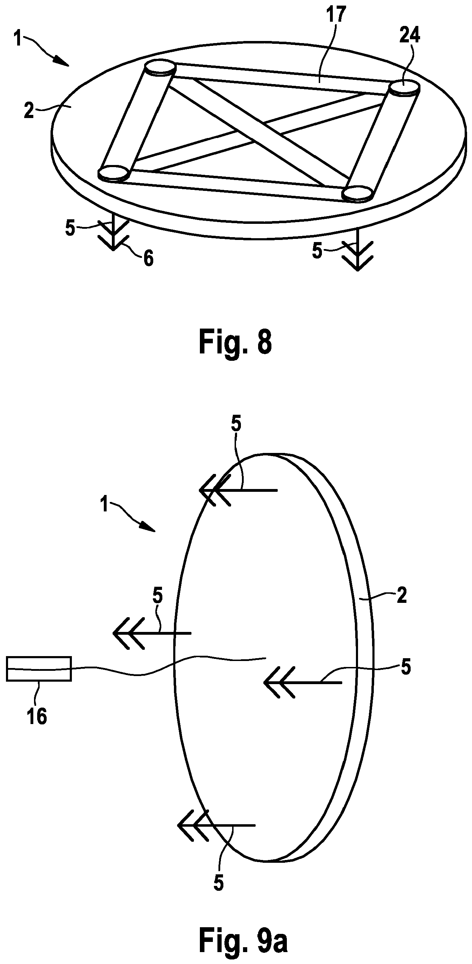

Fig. 8 shows a medical implant that is similar to the implant 1 shown in Fig. 1. Here, the implant further comprises several struts 17 made of biodegradable polyglycolic acid that connect the head areas 24 of the mechanical anchors 5. Thus, a force applied to the medical implant 1 against one of the struts 17 is distributed onto the mechanical anchors 5 and thus facilitates insertion of the anchors 5 into tissue, for example by means of a balloon (see Fig. 1).

Fig. 9a shows an embodiment of a medical implant 1 that comprises a patch 2 with mechanical anchors 5 similar to other described herein. In addition, the medical implant 1 comprises a self-expanding structure 16 made of polyglycolic acid. The self-expanding structure 16 is attached to the patch 2 via a wire 27.

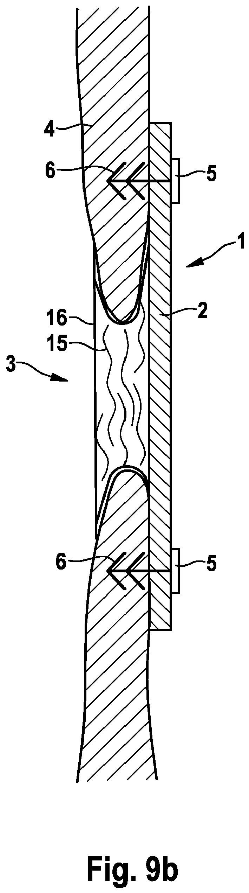

Fig. 9b shows the implant 1 of Fig. 9a in a deployed configuration. The patch 2 is attached to a tissue wall 4 via the mechanical anchors. The self-expanding structure 16 has expanded into a defect 3, here a PFO, and substantially filled the shape of the defect 3. Inside the self-expanding structure 16, thrombogenic fibers 15 are attached that cause a thrombus to form inside the self-expanding structure 16. The thrombus thus occludes the PFO 3.

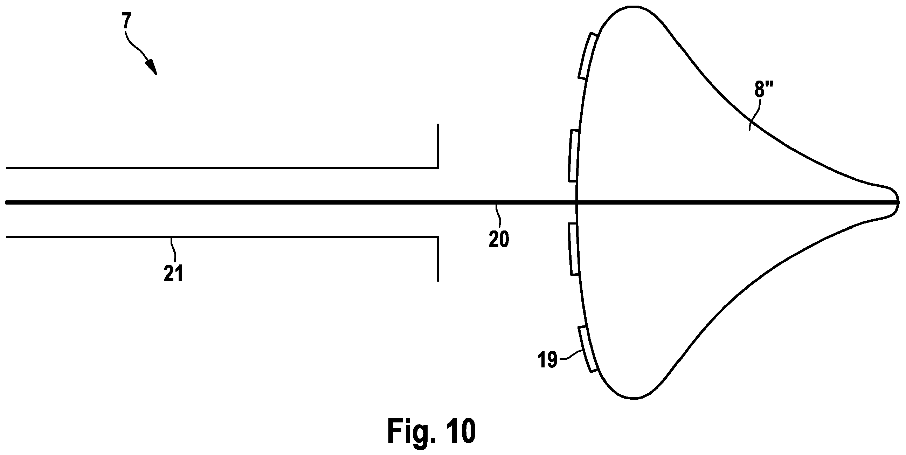

Fig. 10 schematically shows a catheter device 7 adapted to implant a medical implant 1 as shown herein. The catheter device 7 comprises a shaft 3, through which a pull wire 20 extends. The pull wire is made of an implant grade steel, but could alternatively also be a suture, for example made of polyglycolic acid, or a pull wire comprising or consisting of Nitinol. At a distal end of the catheter device 7, an inflatable balloon 8" is arranged and operatively connected to the pull wire 20 and to a conduit for an inflation fluid (not shown). Connectors 19, here made of biocompatible, Velcro-like elements that can temporarily and releasably hold patch (not shown) during implantation. The pull wire 20 is in particular adapted to be pulled such that the balloon 8" exerts a force on the implant (not shown) in order to pierce tissue by means of mechanical anchors.

It will be understood by the person skilled in the arts that any mechanical anchor disclosed herein may be combined with any medical implant, and that combinations of features are merely exemplary embodiments. All features of the mechanical anchors disclosed herein can be combined.

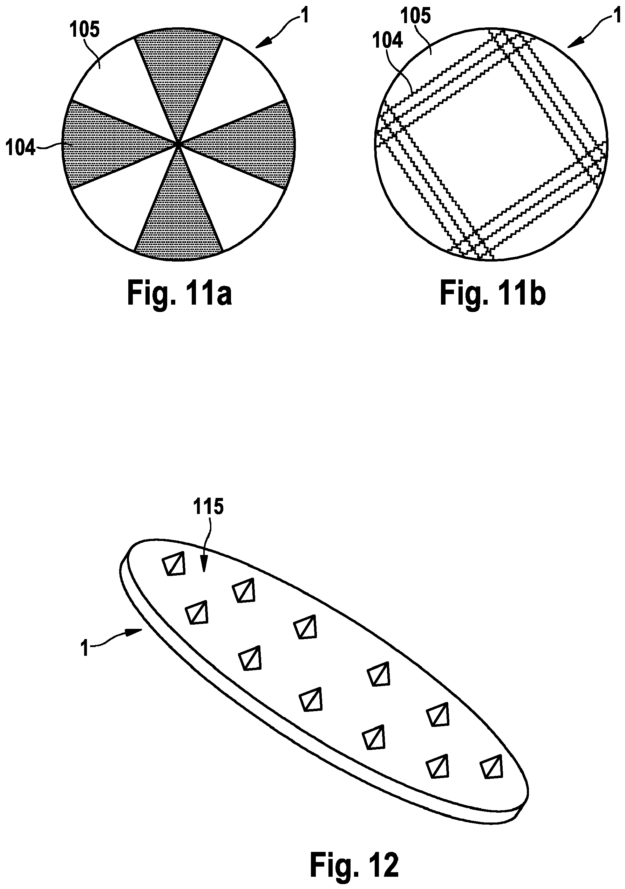

Fig. 11a shows a patch 1 with an adhesive layer 104 that has a patterned structure. The pattern is configured as five sectors of a circular shape that forms the patch 1. Consequently, there are four intermediate sectors 105 that do not comprise an adhesive layer. The adhesive layer 104 was printed via inkjet printing and is based on a mixture of porcine and fish GelMA. Alternatively, extrusion printing may also be employed. Presently, the pattern is two-dimensional. The adhesive layer is thus substantially flat and the sectors 104,105 of the patch 1 differ in whether or not they have an adhesive layer, but not in the thickness of said adhesive layer.

Fig. 11b shows a medical implant similar to the one shown in Fig. 11a. The patch 1 comprises an inkjet-printed pattern of adhesive 104. The adhesive pattern is two-dimensional and is arranged substantially at the circumference of the patch 1. The adhesive pattern comprises several curved lines.

It will be understood that any particular pattern of adhesive may be arranged on a patch, in particular if the adhesive is inkjet printed. Alternatively, extrusion printing may also be employed.

Fig. 12 shows an embodiment of a medical implant 1 with a three-dimensionally patterned adhesive 115. The adhesive is formed as pyramids 116 evenly spaced over one surface of the implant. The medical implant is configured as a patch made of pericardium. The adhesive 115 consists of pyramids 116 of fish GelMA.

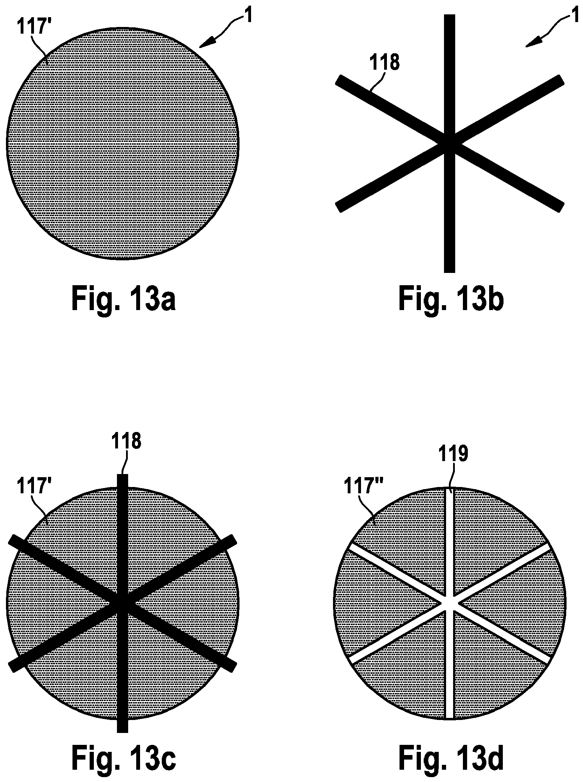

Figs. 13a-13d schematically show a method of patterning an adhesive composition on a medical implant 1.

Fig. 13a shows a medical implant 1 made of polyurethane with a smooth, homogeneous layer 117' of adhesive. The adhesive is based on bovine GelMA.

Fig. 13b shows a stamp-like element 118. The stamp-like element has a shape representing a negative of the desired adhesive pattern on the medical implant 1. The stamp-like element 118 is made of a metallic material with a PTFE coating. Thus, the stamp-like element 118 does not adhere to the adhesive layer 117' and can be removed easily from the adhesive bearing surface.

Fig. 13c shows the stamp-like element 118 being pressed on the adhesive layer 117' of the medical implant 1. The stamp-like element 118 pushes the adhesive laterally away.

Fig. 13d shows the medical implant 1 after removal of the stamp-like element. The adhesive layer 117" has a pattern that substantially corresponds to a negative shape of the stamp-like element. The medical implant 1 hence comprises areas 119 that are not covered by an adhesive layer. The aggregate of the area 119 substantially corresponds to the shape of the stamp-like element.

Any of the implants and adhesives disclosed herein are suitable to be patterned with the method shown in Figs. 13a-13d. It would also be possible to pattern a three-dimensional pattern using the method of Figs. 11a-11b.

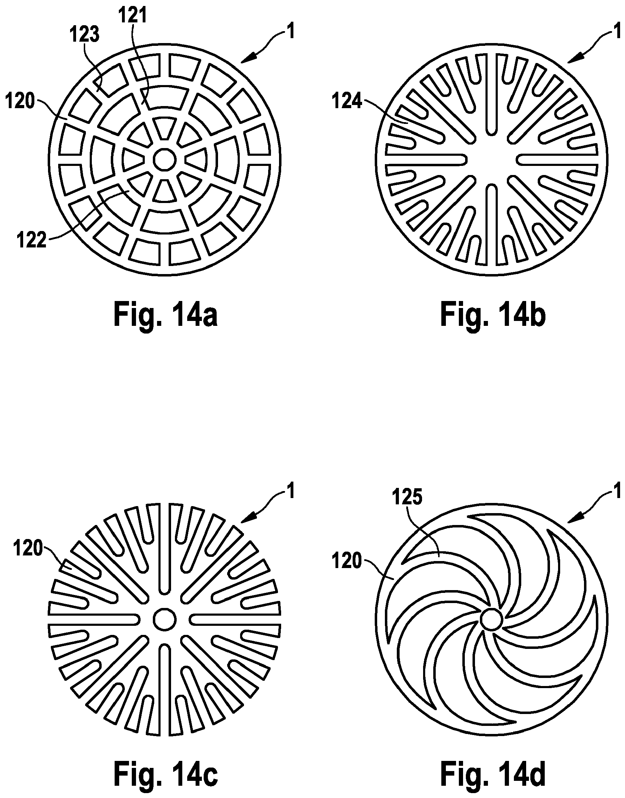

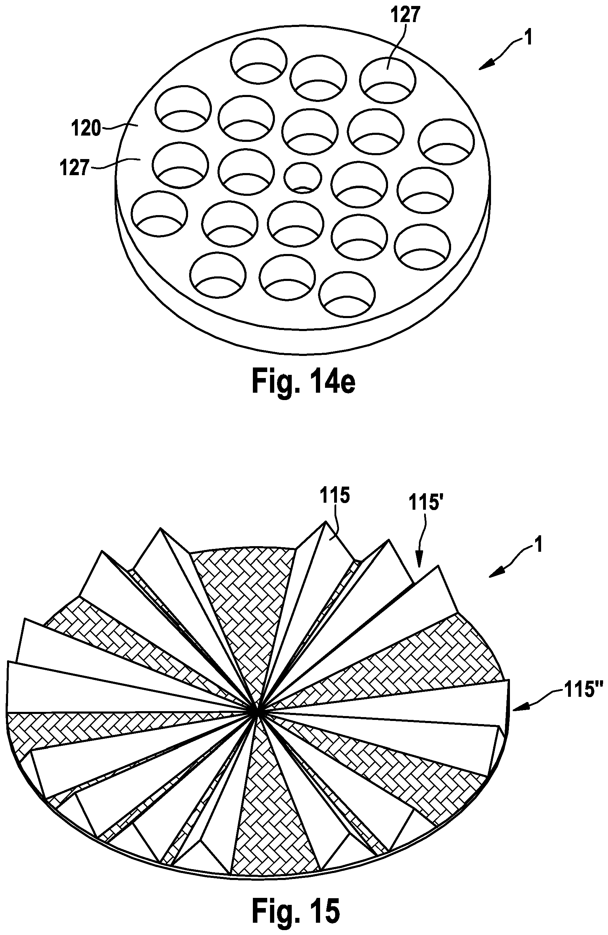

Fig. 14 a shows an embodiment of a two-dimensional adhesive pattern 120 that may be used in combination with any medical device disclosed herein. The pattern 120 comprises radial branches 121 extending through the center of the circular structure. In addition, circular elements 122 are arranged concentrically. In a circumferential area, radially oriented branches 123 are arranged that connect to circular structures.

Fig 14b shows an alternative two-dimensional pattern 120 of adhesive. The pattern 120 comprises radial branches 124 that extend from a circumferential area inwards and are pointed at a center area of the circular structure. The radial branches 124 have varying lengths. It would be conceivable to configure them with constant lengths.

Fig. 14c shows yet an alternative embodiment of a two-dimensional pattern 120 of adhesive. The pattern substantially corresponds to a negative of the pattern shown in Fig. 14b.

Fig. 14d shows an embodiment of a two-dimensional pattern 120 wherein longitudinal elements 125 are configured to extend from a center region toward a circumferential region and comprise a curved area. Here, all the longitudinal elements 125 have the same radius of curvature and are curved uniformly along their length. It would be conceivable to configure different longitudinal elements 125 with different curvatures and/or varying curvatures within one longitudinal element 125.

Fig. 14e shows an embodiment of a two-dimensional pattern 120 of adhesive wherein the adhesive is configured as a layer 126 with holes 127. The arrangement, size, and number of holes 127 may of course vary from the exemplary embodiment shown here.

Fig. 15 shows an embodiment of a three-dimensional pattern of adhesive 115. The adhesive is shaped as radially oriented wedges that extend from a center area to a circumferential area. The wedges have a pyramid-shaped crosssections in a plane perpendicular to the radial extension. The height of the wedge increases toward the circumferential area. The shown embodiment comprises two assemblies 115' comprising three wedges 115 each and arranged on opposite angular sides with respect to the center area. Furthermore, two assemblies 115" comprising two wedges 115 each are arranged between assemblies 115', wherein two assemblies 115" each are arranged opposite to one another with respect to the center area. The pattern 115 is particularly advantageous and suitable for folding the implant within a catheter.

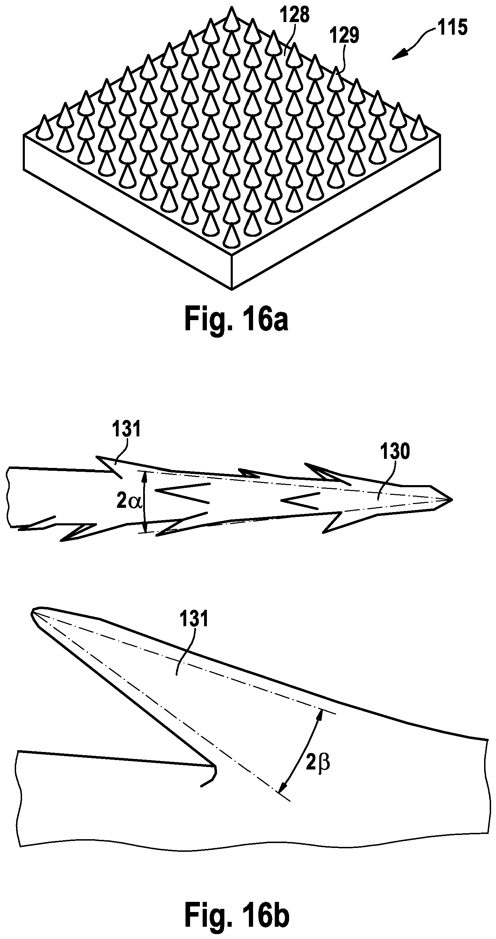

Fig. 16a shows a further embodiment of a three-dimensional pattern 115 of an adhesive. The three-dimensional pattern 115 is configured as a plurality of spikes 128 arranged at uniform distances from one another on a surface 129. Surface 129 may be part of a patch. Here, the surface 129 substantially consists of the same adhesive material as the spikes 128 so as to form a separate patterned adhesive layer that may be arranged on a medical implant (not shown). However, it would be conceivable to arrange the spikes 128 made of the adhesive material on a medical implant without a continuous adhesive layer underneath as well.

Alternatively, spikes 128 may be part of a surface made of a different material, for example a patch material, which is coated with an adhesive to form a substantially similar configuration as shown here.

Fig. 16b shows a possible configuration of a mechanical anchor 130 with barbs 131. Here, the barbs 131 are angled with respect to a longitudinal axis of the mechanical anchor 130 at an angle of α = 35°. The barbs 131 have tips comprising an angle of 2β = 15°, i.e. β =7.5°. It will be understood that any angle α or β between 0 and 90° may be suitable depending on the application. Here, the mechanical anchor 130 is made of an adhesive material and may particularly preferably be combined with the embodiment shown in Fig. 16a, i.e. as a spike 128. However, it will be understood that the shown mechanical anchor 130 may be combined with any other medical implant shown herein and may be configured as mechanical anchor 130 made of an adhesive material, of a non-adhesive material, or any combination thereof, in particular non-adhesive barbs 131 on an adhesive mechanical anchor 130 or vice versa.

## Claims

1. A medical implant (1), preferably comprising a patch (2), that is adapted to close an opening (3), preferably an opening in a ventricular or atrial septum (4), wherein
the medical implant (1) comprises at least one mechanical anchor (5), said anchor preferably comprising at least one barb (6), said anchor being adapted to puncture the patch (2) and/or biological tissue (4) such as to fix the medical implant (1) to said tissue (4),
and is adapted to be deployed by a delivery device (7), in particular a delivery device (7) comprising a balloon (8', 8"), preferably a delivery device (7) comprising at least two balloons (8', 8"), and/or optionally at least one anchor.

2. A medical implant (1) according to one of the preceding claims, wherein the implant (1) comprises an adhesive composition (9), preferably an adhesive composition that is adapted to be curable by exposure to electromagnetic radiation.

3. A medical implant (1) according to one of the preceding claims, wherein the at least one of the mechanical anchor (5) and the implant (1) comprises a magnetic element (10) adapted to connect the medical implant (1) and the mechanical anchor (5) at least partially via a magnetic force.

4. A medical implant (1) according to one of the preceding claims, wherein at least one mechanical anchor (5) comprises at least one cavity (11).

5. A medical implant (1) according to claim 4, wherein the surface of at least one mechanical anchors (5) comprises micro-sized holes (12), in particular pores, that are connected to the at least one cavity (11).

6. A medical implant (1) according to one of the claims 4 or 5, wherein a releasable substance (13) is disposed in at least one cavity (11) of at least one mechanical anchor (5) .

7. A medical implant (1) according to claim 6, wherein the releasable substance (13) comprises an adhesive composition (14), preferably an adhesive composition (14) that is adapted to be curable by exposure to electromagnetic radiation.

8. A medical implant (1) according to one of the claims 6 or 7, wherein the releasable substance (13) comprises a pharmacologically active substance, preferably an anti-inflammatory substance.

9. A medical implant (1) according to one of the preceding claims, wherein the medical implant (1) comprises thrombogenic elements (15).

10. A medical implant (1) according to one of the preceding claim, wherein the mechanical anchors (5) are adapted to reduce their size upon exposure to humidity.

11. A medical implant (1) according to one of the preceding claims, comprising a force distribution structure (17), preferably a force distribution structure (17) consisting of a bio-erodible material, that is connected to the mechanical anchors (5) such that it distributes a mechanical pressure applied to the medical implant (1) among to the among the at least one mechanical anchor (5).

12. A medical implant (1) according to one of the preceding claims, wherein the at least one mechanical anchor (5) comprise at least one needle (18), preferably a micro-needle.

13. The medical implant (1) according to any one of the preceding claims, wherein the mechanical anchor (5) comprises, preferably consists of, an adhesive material.

14. A catheter device, **characterized in that** the catheter device (7) is adapted to deploy a medical device (1) according to one of the claims 1 to 13.

15. A medical implant, preferably a medical implant according to any one of claims 1 to 13, comprising an adhesive, wherein the adhesive is arranged on at least a first surface of the medical implant and formed in a pattern, preferably a two-dimensional pattern or a three-dimensional pattern.

16. The medical implant of claim 15, wherein the pattern comprises at least one, preferably a plurality of, spikes.
